Europäisches Patentamt

European Patent Office

Office européen des brevets

(11) Publication number: **0 360 340**
**A1**

## (12) EUROPEAN PATENT APPLICATION

(21) Application number: 89202332.6

(51) Int. Cl.5: **A61K 9/06** , **A61K 47/00**

(22) Date of filing: 18.09.89

(30) Priority: 19.09.88 FR 8812200

(43) Date of publication of application:
28.03.90 Bulletin 90/13

(84) Designated Contracting States:
AT BE CH DE ES FR GB GR IT LI NL SE

(71) Applicant: AKZO N.V.
Velperweg 76
NL-6824 BM Arnhem(NL)

(72) Inventor: Zirinis, Phedon
38 Avenue Chateau de Bertin
F-78400 Chatou(FR)

(74) Representative: Hermans, Franciscus G.M. et al
Patent Department AKZO N.V. Pharma
Division P.O. Box 20
NL-5340 BH Oss(NL)

(54) Composition for nasal administration containing a peptide.

(57) Composition containing at least one peptide for systemic treatment, the said composition being presented in the form of a nasally administerable lyophilized powder and containing essentially an effective quantity of the said peptide, in combination with a surfactant vehicle.

In its preferred form, the composition is presented in the form of a lyophilized powder containing a pancreatic hormone such as glucagon or insulin. Such a composition is applicable to the treatment of blood sugar disorders.

EP 0 360 340 A1

## Composition for nasal administration containing a peptide

The invention relates to a pharmaceutical composition for systemic treatments, intended for nasal administration, said composition containing at least one peptide which is already known for such treatment but by another administration route. More especially, the invention relates to the field of the treatment of blood sugar disorders, and its subject is especially a new composition suitable for nasal administration and enabling such disorders to be effectively remedied.

It is well known that, for systemic treatments using polypeptides and hormones, the parenteral route (intravenous, intramuscular or subcutaneous) is almost the only one possible, as a result of the degradation of the active substances which would occur with the latter administered orally. Such is the case, for example, with treatments of diseases linked to disorders of carbohydrate metabolism, and especially diabetes. It is known, in effect, that the parenteral administration route is the only one possible for current treatments with insulin, and with glucagon, an insulin antagonist, but such treatment is very restricting for the patients. The latter, rather than receiving a glucagon injection, prefer, in some cases, to take sugar directly by mouth, but this intake subsequently leads to other disorders which have to be treated in order to reestablish the carbohydrate balance.

Similar problems arise for systemic treatments with other medicinal products based on peptides, polypeptides, hormones and the like.

Novel administration routes which are more convenient to use and which, nevertheless, bring about therapeutic results which are as satisfactory as those obtained by the parenteral route, are hence sought. Recently, in a paper in the journal "The Lancet", 18th June 1988, pages 1364 et seq., L. Freychet et al. have described the effect of the intranasal administration of glucagon on the glucose level in the blood of healthy subjects and also that of hypoglycaemic patients suffering from insulin-dependent diabetes. In this publication, the authors have proposed the nasal administration, in spray form, of a solution of glucagon, a known antidote for insulin, in combination with a surfactant agent which, in this instance, is deoxycholic acid. The results obtained show that this nasal administration is effective and could prove advantageous for replacing the parenteral administration of glucagon or of glucose or the oral intake of sugar in the treatment of the blood sugar disorders in insulin-dependent diabetic patients.

However, the presentation in the form of a solution for spraying (spray) of a composition intended for nasal administration is not appropriate to practical requirements. Taking the example of glucagon as an active agent, this substance is known to be incapable of being preserved in solution in an aqueous medium, and in the presence of a surfactant agent. In this case, the problem of presentation becomes paramount. Moreover, the doses of active agent, such as glucagon, administered in spray form are poorly determined and always excessive relative to the truly effective quantity. For example, in the abovementioned publication, the quantity of solution sprayed corresponds to a volume of approximately 0.4 ml, equivalent to 7.5 mg of glucagon per dose, which is in large excess relative to the effective dose. Adverse side effects are also linked to a nasal administration in the form of a sprayed solution, especially an irritation of the nasal mucosa, which can become serious, especially if the treatments are repeated.

The present invention derives benefit from the initial work which was performed on the nasal administration of certain peptides, such as glucagon, in the form of a sprayed solution. It proposes a new form of presentation of a composition capable of being applied by the same route and bringing about equally effective results, while being free from the drawbacks just mentioned. In particular, the new composition is presented in the form of a lyophilized powder which is completely stable over a long period of time.

In its most general form, the invention relates to a composition containing at least one peptide for systemic treatment, the said composition being presented in the form of a nasally administerable lyophilized powder and containing essentially an effective quantity of the said peptide, in combination with a surfactant vehicle.

The invention is applicable to a wide variety of peptides which are capable of being put into the form of a lyophilized powder and exhibit systemic activity after crossing the nasal barrier. As examples of such peptides, reference is made to pancreatic hormones such as glucagon and insulin, and other peptides such as corticotropin (generally designated by the abbreviation ACTH), gonadotrophin or luteinizing hormone (often designated by the abbreviation LHRH) and their derivatives, as well as calcitonin (or thyrocalcitonin) or endorphin and its derivatives, as well as other peptide hormones and their derivatives and all peptides capable of passing through the nasal mucosa in the form of a solution.

In that which follows, the invention will be described more especially in its preferred application, namely the treatment of blood sugar disorders, but it can also be used with peptides specific for the treatment of other diseases or disorders.

Thus, the subject of the invention is, more especially, a composition intended for the systemic treatment of blood sugar disorders, the said composition being presented in the form of a nasally administerable lyophilized powder and containing essentially an effective quantity of at least one pancreatic hormone involved in carbohydrate metabolism, in combination with a surfactant vehicle.

The pancreatic hormone involved in carbohydrate metabolism which participates in such a composition is essentially chosen from glucagon and insulin.

As a substance, glucagon is already known for its hyperglycaemic activity (see The Merck Index, 9th edition, pages 573 and 574, compound no. 4277). The difficulties of presentation of a composition containing glucagon are, in particular, linked to the fact that this substance is almost insoluble in water and that it is soluble only in an acidic or basic medium, at pH values below 3 or above 9.5, respectively.

Insulin is a hormone having hypoglycaemic activity, it is widely used in the treatment of diabetes (see also The Merck Index, 9th edition, page 659 no. 4859). While this substance is readily soluble in dilute bases and acids, it is almost insoluble in an aqueous medium in a pH range from 4.5 to 7.

The other component of the composition according to the invention is a surfactant vehicle. This vehicle fulfils a dual function. In the first place, it enables a solution of the active agent to be produced under good conditions, before the lyophilization designed to yield the final form of the composition, which is a powder. In the second place, the vehicle serves to cross the nasal barrier when the powder is applied on the nasal mucosa. By routine tests, those versed in the art can choose a suitable surfactant vehicle, which can consist of a single surfactant substance or a mixture of such substances. By way of non-limiting examples, surfactant products suitable as vehicles in the composition of the invention, which is capable of being administered nasally for systemic treatment, are as follows, alone or mixed:

Sodium cholate
Sodium deoxycholate
Sodium glycocholate
Sodium dehydrocholate
Sodium glycodeoxycholate
Sodium taurodeoxycholate
Polyoxyethylene (9) lauryl ether
Polyoxyethylene (10) cetyl ether
Sodium taurodihydrofusidate

The relative proportions of the surfactant vehicle and of the active agent in the composition of the invention can vary within wide limits. Thus, the surfactant/active agent ratios are generally between 1:0.5 and 1:4 by weight, the best results having been obtained with substantially equal quantities by weight of vehicle and active agent.

According to the invention, it is very important that the composition is presented in the form of a lyophilized powder as such. In effect, such a powder is hygroscopic and, during nasal administration, it is applied on the nasal mucosa, in contact with which it gradually dissolves. Conversely, a non-lyophilized powder which contains up to 10% of residual moisture does not permit the active agent to pass effectively through the nasal mucosa. The lyophilization treatment hence enables the active agent to be combined with the surfactant vehicle within a homogeneous powder, which is made hygroscopic and is thus suitable for intranasal administration.

It was also found, as will be shown below, that the new form of presentation according to the invention enabled doses of active agent to be used which were markedly smaller in quantity than in the case of a presentation in the form of a solution for spraying. For example, in the case of glucagon, a 1 mg quantity of active agent in the lyophilized powder of the invention gives the same results as a 7.5 mg quantity in a dose of sprayed solution. This result is extremely advantageous, since the invention makes it possible not only to reduce consumption of medicinal product, but also to dose more satisfactorily the quantity of active agent administered.

It is advisable to store the composition according to the invention in dosage units, such as small tubes, sealed so as not to permit the entry of moisture. It is necessary, in effect, to secure the hygroscopic nature of the lyophilized pulverulent composition. The doses to be used will depend on the active agent participating in the composition. For example, in the case of glucagon, dosage units containing 1 mg and 2 mg, respectively, of glucagon have given good results. In the case of insulin, the dosage units can contain, respectively, 5, 10, 50 or 100 units of this hormone.

For the manufacture of the composition according to the invention, it is possible to proceed as follows. In a first stage, an aqueous solution of the active agent, such as glucagon or insulin, must be prepared by mixing the chosen surfactant vehicle with the agent, under pH conditions determined according to the nature of this vehicle. For example, in the case of glucagon, the active agent is first dissolved in sodium hydroxide to pH 9.5, after which an aqueous solution of the surfactant vehicle is added, the pH being finally adjusted to 9.5 if necessary. For surfactant vehicles which are soluble in an acid medium, the procedure will be performed at acid pH, for example in the region of 3, producing the solution of glucagon at this pH by adding hydrochloric acid, the aqueous solution of surfactant being produced under the same conditions. In the case of insulin, the latter, in the desired quantity, is first dissolved in dilute hydrochloric acid until a pH of 2.5 is attained, after which sodium hydroxide is added to attain a pH of 7.3. The surfactant vehicle is introduced in aqueous solution in a corresponding quantity and the pH is finally adjusted to 7.3. For surfactant vehicles soluble at acid pH, the procedure is performed directly in this pH region, for example from 2.5 to 3, without adding sodium hydroxide.

In all cases, a homogeneous aqueous solution containing the active agent and the surfactant vehicle is obtained, and it is advisable to subject this rapidly to lyophilization on account of the poor properties of preservation, in particular beyond 24 hours. For the purpose of lyophilization, the solution is preferably distributed into dosage units. The lyophilized powder obtained is - preferably after drying - used directly for the purpose of nasal administration. For example, it is possible to use tubes having a volume of 0.2 ml. The actual lyophilization begins with rapid freezing at low temperature, especially below -30° C and preferably of the order of -40° C, for a period not exceeding 15 hours, for example of the order of 12 hours. Lyophilization is then effected under high vacuum, for example of the order of 0.05 mbar for a period of 24 hours. Finally, the lyophilisates are dried under vacuum without exceeding a temperature of 15° C. After this drying, the dosage units are sealed with protection from moisture. Thus, the tubes should be stoppered immediately after lyophilization.

For the purpose of administration, the dosage unit, such as a tube, is opened and the powder is applied on the nasal mucosa by any suitable means. In some cases, it would be sufficient to place the quantity of powder on the back of the hand and to inhale it through the nose, in the manner of a pinch of snuff. It is nevertheless preferable to connect to the tube containing the powder a length of tubing the other end of which can be placed in the mouth to blow and expel the powder into the nose. These are only simple examples of use of the composition according to the invention, no limitation being implied.

In the foregoing, the invention has been described by providing detailed information about the essential components of the lyophilized powder composition, namely a peptide, and especially a pancreatic hormone involved in carbohydrate metabolism, for example glucagon or insulin, and the surfactant vehicle. Naturally, the lyophilized powder may if desired contain other active agents and adjuvants which do not fundamentally modify its function but permit better use or easier nasal application. Examples of such adjuvants are lactose, diethylene glycol monoethyl ether, chlorobutanol and the like. In another aspect, the invention hence also relates to a composition of the type mentioned above, containing an active agent, as defined, in combination with a surfactant vehicle and/or an adjuvant as has just been described.

The invention will be further illustrated, without being in any way limited, by the examples below.

EXAMPLE 1

Preparation of a composition based on glucagon in the form of a lyophilized powder for intranasal administration.

10 and 20 mg quantities of glucagon, respectively, were used. They were treated with 1 ml of 0.001 N sodium hydroxide until a pH of 9.5 was attained. An aqueous solution containing 10 and 20 mg/ml, respectively, of sodium taurodihydrofusidate as surfactant agent was then added. The pH of the solution was then again adjusted to 9.5 by adding sodium hydroxide.

The solution prepared as above was then distributed, in 0.2 ml portions, into tubes arranged for nasal administration. Each tube was immediately frozen at -40° C for 12 hours. Lyophilization was then performed under a vacuum of 0.05 mbar for 24 hours. Finally, the lyophilisates were dried for 2 hours under vacuum without exceeding a temperature of 15° C. The tubes containing the lyophilized powder were immediately stoppered after lyophilization.

EXAMPLE 2

The procedure was as in Example 1, using other surfactant vehicles and replacing sodium taurodihydrofusidate by equivalent quantities of sodium glycocholate.

The procedure followed was then as before, until tubes containing the lyophilized powder were obtained.

EXAMPLE 3

In this example, a surfactant soluble in acid medium was employed, namely polyoxyethylene (9) lauryl ether, in which case the glucagon solution was produced in an acid medium at pH 3 using doses of 10 mg and 20 mg, respectively, of glucagon in 1 ml of 0.01 N hydrochloric acid at pH 3. The surfactant, which was used in aqueous solution containing 10 and 20 mg/ml, respectively, was treated likewise, also working at pH 3.

The actual lyophilization was performed in the same manner as in Examples 1 and 2.

## EXAMPLE 4

The procedure was as in Example 3, replacing polyoxyethylene (9) lauryl ether by another surfactant soluble in acidic medium, such as polyoxyethylene (10) cetyl ether.

The lyophilized powder in tubes suitable for intranasal administration was also obtained.

## EXAMPLE 5

In this example, a lyophilized powder based on insulin, suitable for nasal administration, was prepared.

Quantities of 5, 10, 50 or 100 units of insulin, respectively, dissolved in 0.01 N dilute hydrochloric acid solution to pH 2.5, were used. 0.1 N sodium hydroxide was then added to pH 7.3. To this insulin solution, 1, 10, 20 or 200 mg quantities, respectively, of an aqueous solution of sodium glycocholate were added by way of a surfactant. After mixing, the pH was checked and adjusted to 7.3. The mixtures were finally made up with water to a total quantity of 20 ml of solution, which was distributed into tubes intended for lyophilization. The latter was carried out as in Examples 1 to 4 above. A lyophilized powder based on insulin, suitable for nasal administration, was thereby obtained.

## EXAMPLE 6

The procedure was as in Example 5, but replacing sodium glycocholate by equivalent quantities of another surfactant such as sodium deoxycholate. Dosage units containing lyophilized powder based on insulin were also obtained.

Clinical experiments

Clinical experiments were undertaken in order to illustrate the activity of the composition according to the invention, and the latter was compared with a composition presented in the form of a solution for spraying nasally, as described in the abovementioned publication The Lancet of 18th June 1988.

In these trials, a lyophilized powder according to Example 1, containing 1 mg of glucagon as active agent and, by way of a surfactant vehicle, 1 mg of sodium taurodihydrofusidate, was used. The solution for comparison, for spraying, contained 7.5 mg of glucagon in the presence of deoxycholic acid as surfactant, in the proportion of 1% weight/volume.

The working protocols are the same as those described in the publication The Lancet, and hence do not need to be restated in detail. In summary, trials were first undertaken on non-diabetic, healthy volunteers. These volunteers, fasted since the previous day, were treated on the following morning with a dose of the intranasal composition according to the invention. Beforehand, each volunteer had received a venous catheter for withdrawal of samples. After the nasal administration, the levels of glucose and of glucagon, respectively, in the blood are measured at frequent intervals, up to 30 minutes. Similar trials were undertaken on diabetic subjects suffering from insulin-induced hypoglycaemia.

The results obtained are collated on the diagrams in the attached drawings, wherein:

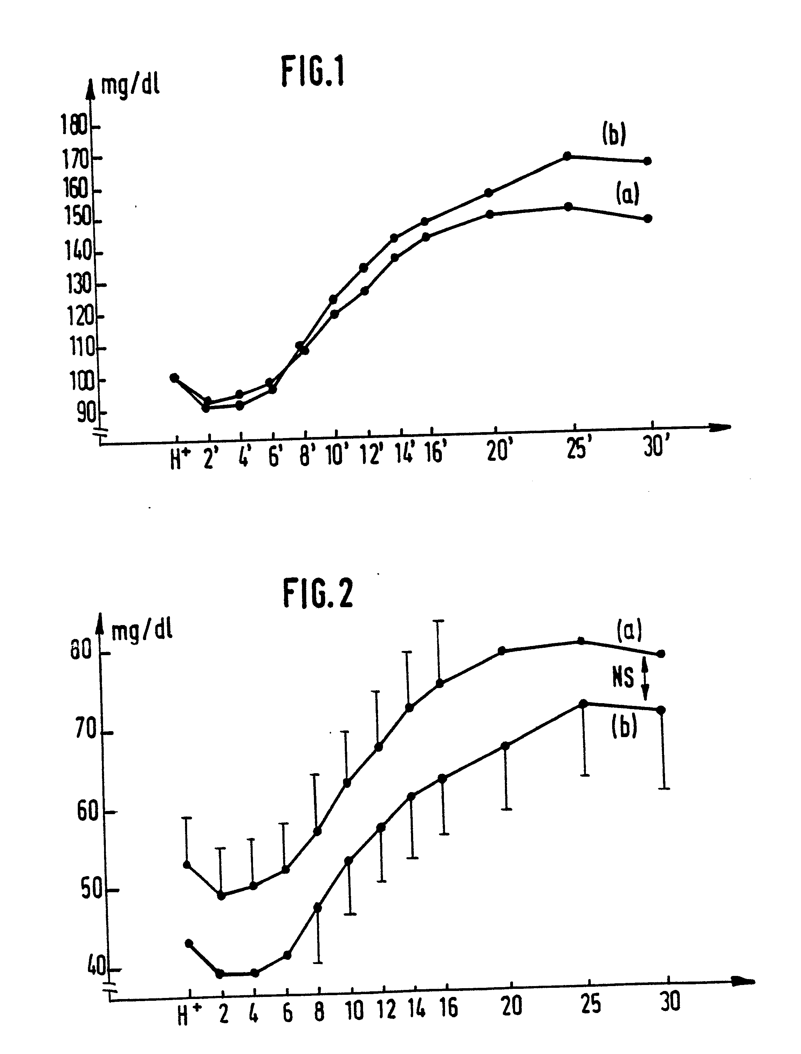

Fig. 1 shows the curves (a) and (b), respectively, obtained by plotting as ordinates, in mg/dl, the quantity of glucose in the blood of the non-diabetic volunteers, and as abscissae the times at which the measurements were made after administration of the composition, the curve (a) corresponding to the composition according to the invention (1 mg of glucagon in lyophilized powder) and the curve (b) to the sprayed solution according to the prior art (7.5 mg of glucagon in solution). In all cases, the number of volunteers was equal to 6.

Fig. 2 shows the analogous curves obtained by measurement of the glucose in the blood of diabetic patients suffering from insulin-induced hypoglycaemia. The curve (a) corresponds to the results obtained with the composition according to the invention, and the curve (b) to those yielded by a composition in the form of a sprayed solution. In this case also the number of subjects treated was equal to 6. The means and the standard deviations, in accordance with the standardized methods of measurement as described in the abovementioned publication The Lancet, are shown on the figure.

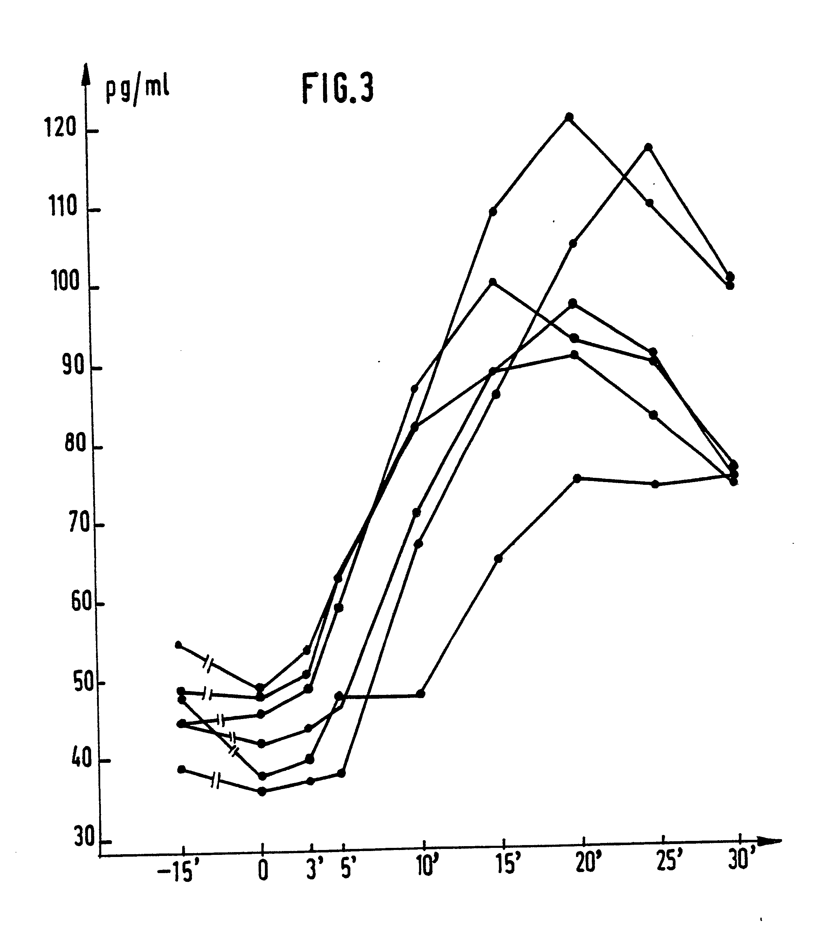

Fig. 3 is a diagram in which the curves for the measurements obtained on six healthy subjects in the same manner as in Figures 1 and 2 have been collated, but in this case the measurements made and plotted as ordinates corresponded to the level of glucagon in the blood, expressed in pg/ml, the said subjects having received at time 0 the administration of a dose of composition in the form of lyophilized powder according to the invention.

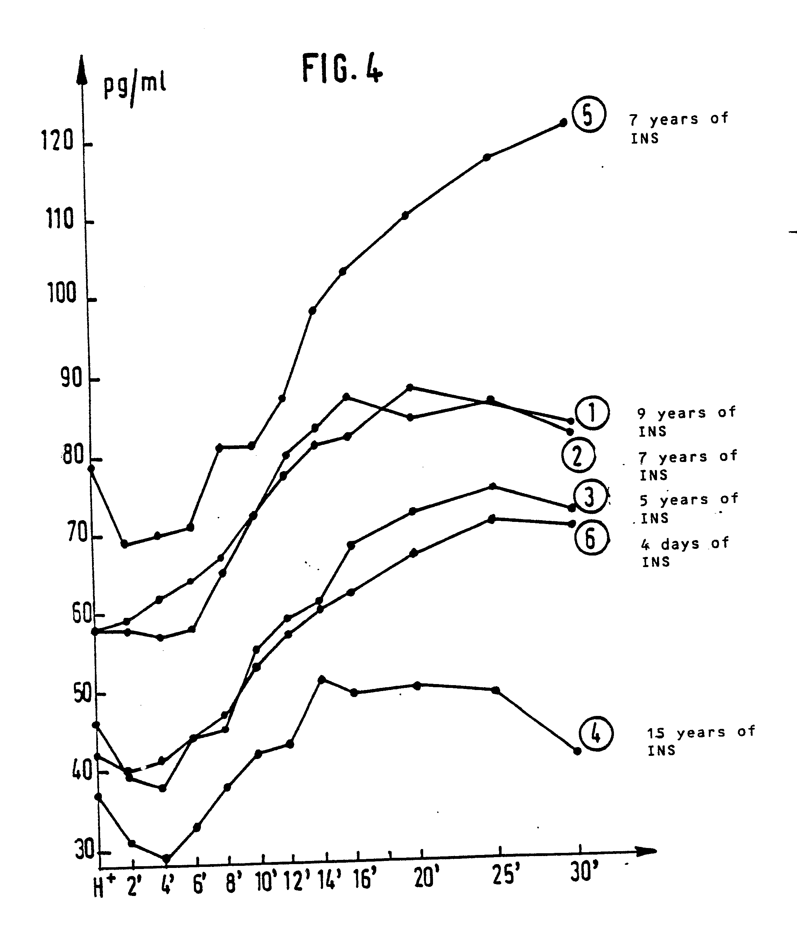

Fig. 4 is a diagram similar to Figure 3, but in which the measurements obtained on diabetic patients suffering from insulin-induced hypoglycaemia have been plotted. Each of the six patients received at time 0 a dose of the composition according to the invention in the form of a nasally administered lyophilized powder. The legends accompanying each of the curves in Figure 4 relate to the diabetic patient's past, and characterize the period of treatment with insulin.

The results illustrated in attached Figures 1 to 4 clearly demonstrate the efficacy of the composition according to the invention. After the administration of a dose of lyophilized powder containing glucagon, the glucose level increases markedly in the blood, especially in the first twenty minutes following administration, both in healthy subjects and in diabetic patients. It is also found that the results recorded are at least as good as those obtained with a sprayed solution containing 7.5 mg of glucagon, whereas the powder composition according to the invention contains only one mg thereof.

The same observations may be made regarding the diagrams illustrating the measurements of the level of glucagon in the blood. The values obtained demonstrate that the nasal administration of a lyophilized powder composition containing glucagon is an effective means for making this active agent available for a systemic treatment.

Other clinical trials were undertaken on healthy volunteers, according to the same working protocols as those described above for glucagon, but on this occasion with two compositions based on insulin:
- the first (1) containing 5 units of insulin and 1 mg of sodium taurodihydrofusidate as surfactant,
- the second (2) containing 5 units of insulin and 1 mg of glycocholic acid (sodium glycocholate) as surfactant.

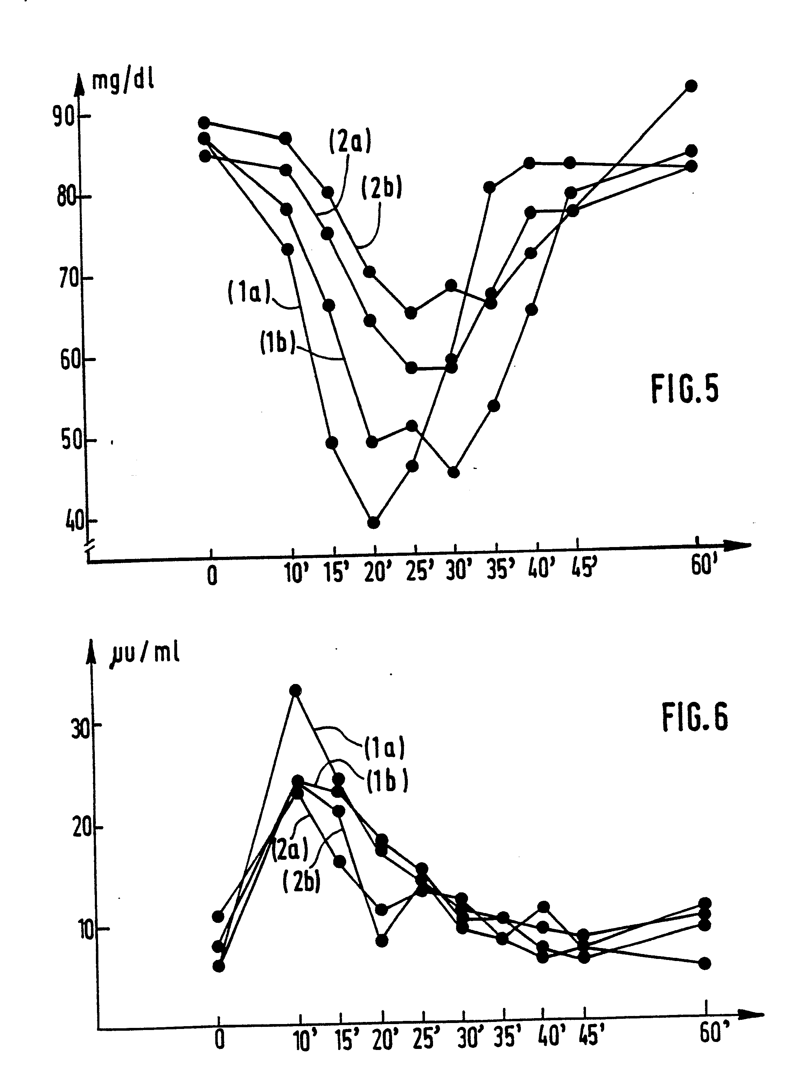

The results obtained are illustrated in Figures 5 to 6:

Figure 5 is a diagram showing the curves (1a), (1b) and (2a), (2b) obtained on two patients, plotting as ordinates, in mg/dl, the quantity of glucose in the blood (blood sugar level) and as abscissae the times at which the measurements were made after administration at time 0 of the compositions (1) and (2) above, respectively.

Figure 6 is an analogous diagram in which the level of insulin (blood insulin level) in $\mu$U/ml in the blood has been plotted as ordinates, and the times at which the measurements were made after administration at time 0 of the compositions (1) and (2) above, respectively, have been plotted as abscissae.

These results demonstrate that the pharmaceutical dosage form of the insulin lyophilisate containing approximately 0.1 U/kg, in combination with a surfactant, lowers the blood sugar level as much as the same quantity of insulin injected intravenously or subcutaneously.

This action is also reflected in the plasma insulin level, which rises from the 10th minute from 10 to 30 $\mu$U/ml.

## Claims

1. Composition containing at least one peptide for systemic treatment, the said composition being presented in the form of a nasally administerable lyophilized powder and containing essentially an effective quantity of the said peptide, in combination with a surfactant vehicle.

2. Composition according to Claim 1, characterized in that the peptides are suitable for being put into the form of lyophilized powder and exhibit systemic activity after crossing the nasal barrier, and are, for example, pancreatic hormones such as glucagon and insulin, or other peptides such as corticotropin (generally designated by the abbreviation ACTH), gonadotrophin or luteinizing hormone (often designated by the abbreviation LHRH) and their derivatives, as well as calcitonin (or thyrocalcitonin) or endorphin and its derivatives, as well as other peptide hormones and their derivatives and all peptides capable of passing through the nasal mucosa in the form of a solution.

3. Composition intended for the systemic treatment of blood sugar disorders, the said composition being presented in the form of a nasally administerable lyophilized powder and containing essentially an effective quantity of at least one pancreatic hormone involved in carbohydrate metabolism, in combination with a surfactant vehicle.

4. Composition according to Claim 3, characterized in that the pancreatic hormone is glucagon or insulin.

5. Composition according to any one of Claims 1 to 4, characterized in that the surfactant vehicle is chosen from the following compounds, alone or mixed:
Sodium cholate
Sodium deoxycholate
Sodium glycocholate
Sodium dehydrocholate
Sodium glycodeoxycholate
Sodium taurodeoxycholate
Polyoxyethylene (10) cetyl ether
Polyoxyethylene (9) lauryl ether
Sodium taurodihydrofusidate

6. Composition according to any of Claims 1 to 5, characterized in that the surfactant/active agent ratios are between 1:0.5 and 1:4 by weight, and are, in particular, 1:1 by weight.

7. Composition according to any one of Claims 1 to 6, presented in the form of sealed dosage units.

8. Composition according to Claims 4 and 7, characterized in that each dosage unit contains 1 or 2 mg of glucagon.

9. Composition according to Claims 4 and 7, characterized in that each dosage unit contains 5, 10, 50 or 100 units of insulin.

10. Composition according to any one of Claims 1 to 9 containing a lyophilized active agent, as defined, in combination with a surfactant vehicle and/or an adjuvant facilitating the use and nasal application.

11. Composition according to Claim 10, characterized in that the adjuvant is, for example, lactose, diethylene glycol monoethyl ether, chlorobutanol and the like.

12. Process for obtaining a composition according to any one of Claims 1 to 11, characterized in that, after a solution of the active agent and the surfactant vehicle and/or the adjuvant having a suitable pH has been produced, the said solution is immediately subjected to a lyophilization comprising the following stages:
- rapid freezing at low temperature, below -30°C, and preferably of the order of -40°C, for a period not exceeding 15 hours, for example of the order of 12 h,
- lyophilization under high vacuum, for example of the order of 0.05 mbar, for 24 h,
- drying of the lyophilisates under vacuum without exceeding a temperature of 15°C.

13. Process according to Claim 12, characterized in that the solution is packaged in dosage units before being subjected to lyophilization, and in that, when the latter is complete, the said units are sealed with protection from moisture.

14. Application of the composition according to one of Claims 1 and 2, or obtained by the process according to one of Claims 12 and 13, to the treatment of diseases or disorders involving an administration of at least one specific peptide as defined.

15. Application of the composition according to any one of Claims 3 to 9, or obtained by the process according to one of Claims 12 and 13, to the systemic treatment of blood sugar disorders.

## FIG.1

## FIG.2

FIG.3

FIG. 4

FIG.5

FIG.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | STN International Information Services Data Base: Chemical Abstracts, Accession Number 110 (2):13573d; & JP-A-63 002 932 (TEIJIN LTD) 07-01-1988 * Abstract * | 1-15 | A 61 K    9/06 A 61 K   47/00 |
| X | EP-A-0 193 372  (TEIJIN LTD.) * claims 1-6,9-10,12-13; column 2, lines 61-66; column 3, lines 11-15,24-34; column 4, lines 46-55; column 5, lines 35,45-52 * | 1-15 | |

TECHNICAL FIELDS
SEARCHED (Int. Cl.5)

A 61 K

The present search report has been drawn up for all claims

| Place of search | Date of completion of the search | Examiner |
|---|---|---|
| THE HAGUE | 18-10-1989 | SCARPONI U. |